# EUROPEAN PATENT APPLICATION

(11) **EP 4 837 625 A1**
(43) Date of publication of application: **11.11.2026**
(21) Application number: 24915483.2
(22) Date of filing: 17.12.2024
(51) Int. Cl.: A61N 1/40, A61N 1/08, A61N 1/06, A61N 1/32

(54) **TREATMENT DEVICE USING RF ENERGY, HANDPIECE THEREOF, AND TIP MODULE THEREOF**

(30) Priority: 04.01.2024 KR 20240001509
(71) Applicant: Lutronic Corporation, Goyang-si, Gyeonggi-do 10534 (KR)
(72) Inventor: KO, Kwangchon, Goyang-si, Gyeonggi-do 10394 (KR)
(74) Representative: Algemeen Octrooi- en Merkenbureau B.V.
(86) International application number: PCT/KR2024/097062
(87) International publication number: WO 2025/147073

(57) **Abstract**

The present disclosure relates to a treatment apparatus using RF energy, a handpiece of the treatment apparatus, and a tip module of the treatment apparatus. There are provided: a treatment apparatus using RF energy including an electrode configured to transmit RF energy to tissues, and a cooling module provided behind the electrode and including a plurality of branched channels, through which a refrigerant is delivered, and a plurality of spray holes provided at ends of the channels, respectively, to discharge the refrigerant, wherein the branched channel has a shape of which a diameter increases in a flow direction of the refrigerant in a section adjacent to the spray hole; a handpiece of the treatment apparatus; and a tip module of the treatment apparatus.

## Description

### TECHNICAL FIELD

The present disclosure relates to a treatment apparatus using RF energy, a handpiece of the treatment apparatus, and a tip module of the treatment apparatus.

### BACKGROUND

Technologies of transmitting RF energy to tissues to treat lesions have been developed in various ways. In particular, recently, technologies of treating tissues without damaging the skin surface by performing treatment by transmitting RF energy while cooling the skin with electrodes in contact with the skin surface have been developed. Such treatment technologies using RF energy have been disclosed in Korean Patent No. 0706115, etc.

A cooling structure that cools an electrode during treatment is provided to prevent overheating of the electrode in such treatment using RF energy. However, cooling structures of the related art have difficulty in uniform cooling of an electrode.

### SUMMARY

An objective of the present disclosure is to provide: a treatment apparatus using RF energy that can uniformly cool an electrode when cooling the electrode in consideration of the heat generation characteristic that depends on the position of the electrode in treatment of tissues using RF energy; a handpiece of the treatment apparatus; and a tip module of the treatment apparatus.

In order to achieve the objectives of the present disclosure, the present disclosure provides a treatment apparatus using RF energy that includes: an electrode configured to transmit RF energy to tissues; and a cooling module provided behind the electrode and including a plurality of branched channels, through which a refrigerant is delivered, and a plurality of spray holes provided at ends of the channels, respectively, to discharge the refrigerant, wherein the branched channel has a shape of which a diameter increases in a flow direction of the refrigerant in a section adjacent to the spray hole.

The section of the branched channel that is adjacent to the spray hole forms an inner wall inclined outward from a center axis of the channel.

The inclined inner wall has an inclination angle within a range of 10° to 80° from the center axis of the channel.

The cross-sectional area of the spray hole is 1.2 or more times a cross-sectional area of the channel before the section of which the diameter increases in the branched channel.

A plurality of spray holes are provided on a surface of the cooling module and the plurality of spray holes are symmetrically disposed on the surface of the cooling module. As an example, at least four or more spray holes may be provided on the surface of the cooling module. The plurality of branched channels are formed to have the same length.

The plurality of spray holes are disposed at positions spaced a predetermined distance radially from a center of a surface of the cooling module and the spaced distance may be a half or more of a distance from a center to an outer edge of the surface of the cooling module.

Meanwhile, the branched channel has a widening section formed such that a diameter increases in the flow direction of the refrigerant at a position adjacent to the spray hole, and an inner wall forming the widening section is formed to have different inclination angles.

In this configuration, in the inner wall forming the widening section, the inner wall close to a center of a surface of the cooling module may have a larger inclination angle than the inner wall far from the center of the surface of the cooling module.

Alternatively, the treatment apparatus may further include a distributing member disposed between the electrode and the cooling module and configured to distribute the refrigerant discharged through the spray holes.

Meanwhile, the objectives of the present disclosure described above may be achieved by a handpiece or a tip module of the treatment apparatus using RF energy, the handpiece or the tip module including: an electrode configured to transmit RF energy to tissues; and a cooling module provided behind the electrode and including a plurality of branched channels, through which a refrigerant passes, and spray holes provided at ends of the channels, respectively, to discharge the refrigerant, wherein the branched channel has a shape of which a diameter increases in a flow direction of the refrigerant in a section adjacent to the spray hole.

According to the present disclosure, even though a refrigerant is sprayed toward the outer side of an electrode to cool the electrode, even the center portion of the electrode is sufficiently cooled, so the electrode can be uniformly cooled and damage to skin during treatment can be prevented.

### BRIEF DESCRIPTION OF THE DRAWINGS

FIG. 1 is a perspective view showing a treatment apparatus using RF energy according to an embodiment of the present disclosure.
FIG. 2 is a block diagram showing the main configuration of the treatment apparatus shown in FIG. 1.
FIG. 3 is a perspective view showing a handpiece shown in FIG. 1.
FIG. 4 is an exploded perspective view showing the main configuration of the tip module shown in FIG. 3.
FIG. 5 is a cross-sectional view showing the main configuration of the tip module shown in FIG. 3.
FIG. 6 is a perspective view showing an embodiment of the cooling module shown in FIG. 4.
FIG. 7 is a plan view showing the front surface of the cooling module.
FIG. 8 is a cross-sectional view showing a cross-section of the cooling module according to an embodiment.
FIG. 9 is a cross-sectional view showing a cross-section of a cooling module according to another embodiment.
FIG. 10 is a cross-sectional view showing a cross-section of a cooling module according to another embodiment.
FIG. 11 is a cross-sectional view showing a cross-section of a cooling module according to another embodiment.
FIG. 12 is a cross-sectional view showing a cross-section of a cooling module according to another embodiment.

### DETAILED DESCRIPTION

Hereafter, a treatment apparatus using RF energy according to an embodiment of the present disclosure, a handpiece of the treatment apparatus, and a tip module of the treatment apparatus are described in detail with reference to the drawings. In the following description, the positional relationships of components are principally explained with reference to the drawings. Further, the drawings may simplify the structure of the present disclosure or may be exaggerated, if necessary, for the convenience of description. Accordingly, the present disclosure is not limited thereto and it should be understood that various devices may be added, modified, or omitted.

Hereafter, a "treatment apparatus using RF energy" includes all apparatuses for treating mammals, including humans. A treatment apparatus may include various apparatuses that perform treatment by transmitting RF energy for the purpose of improving the conditions of lesions or tissues. The following embodiment will be described with a focus on an apparatus for treating skin lesions. For example, the apparatus may mean apparatuses that achieve effects such as improvement in wrinkles, tone and textural changes, scars and acne scarring, sagging mucosa, overall rejuvenation, hyperhidrosis laxity, lifting, tightening, and fat reduction by locally heating skin tissues using RF energy. However, it should be noted that the present disclosure is not limited thereto and can be applied to various apparatuses that are used to transmit RF energy to various affected areas, including apparatuses that surgically treat internal organ lesions.

Hereafter, a "tissue" refers to a group of cells constituting various body organs of animals, including humans. The "tissue" includes the skin tissues of the face, neck, arms, legs, and torso, as well as various tissues constituting various internal organs.

Hereafter, a treatment apparatus using RF energy according to an embodiment of the present disclosure is described with reference to the drawings.

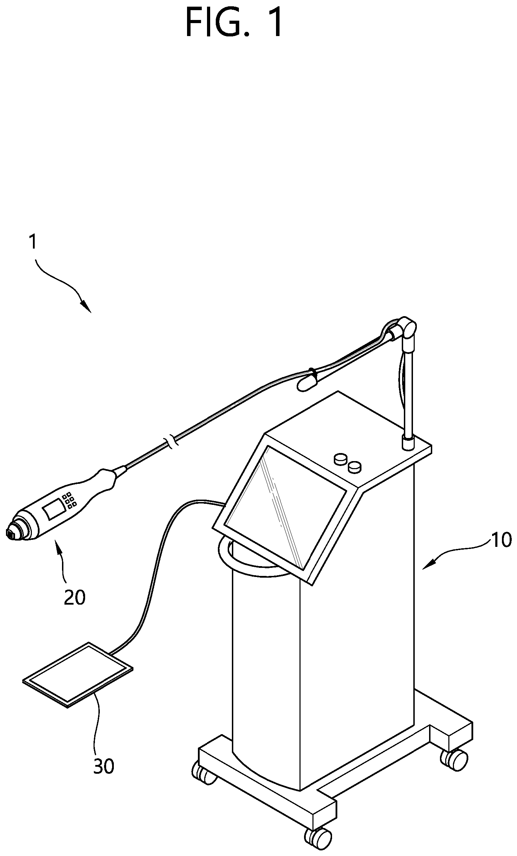

FIG. 1 is a perspective view showing a treatment apparatus using RF energy according to an embodiment of the present disclosure.

As shown in FIG. 1, a treatment apparatus using RF energy according to this embodiment includes a body 10, a handpiece 20 connected with the body 10, and a return electrode pad 30.

The body 10 has various components for operating the treatment apparatus of the embodiment. Various switches and a display unit for setting/performing operations of the treatment apparatus are disposed on the outer surface of the body 10. Further, components such as an RF energy generator 50 and a refrigerant storage may be provided in the body 10.

The handpiece 20 is a component for performing treatment at treatment positions and is designed to be held and used by a user. An electrode 60 that transmits RF energy in contact with the skin surface of a patient is disposed at an end of the handpiece 20. Various controls for operating treatment may be disposed on the outer surface of the handpiece 20, and a conductive path for transmitting RF energy to the electrode 60 and a cooling channel for cooling the electrode are provided in the handpiece 20.

The return electrode pad 30 includes a return electrode. The return electrode is made of a conductive material and is positioned to contact a location on the patient's body opposite a treatment position during treatment. Accordingly, when RF energy is applied, the return electrode forms a path through which RF energy is transmitted to the patient's body together with the electrode of the handpiece.

As shown in FIG. 1, the handpiece 20 and the return electrode pad 30 are connected to the body by connectors, respectively. The connectors may be cables, etc., and each of the connectors is electrically connected to the body, whereby it forms an RF transmission path and is configured to be able to transmit or receive various signals.

In the embodiment, the electrode 60 of the handpiece is configured in a monopolar type having one polarity, so a separate return electrode pad is included, but the present disclosure is not limited thereto. As another example, when the electrode 60 of the handpiece is configured in a bipolar type having different polarities, the return electrode pad described above may not be included.

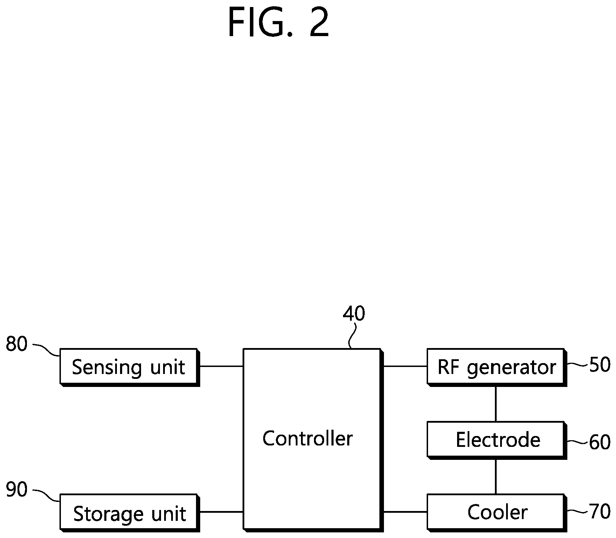

FIG. 2 is a block diagram showing the main configuration of the treatment apparatus shown in FIG. 1.

The RF energy generator 50 generates RF energy that is used for treatment. The RF energy generator 50 generates RF pulses having various parameters, depending on the patient's condition, treatment purpose, treatment area, etc. The parameters may be at least one of output, pulse duration, pulse interval, and frequency. The RF energy generated by the RF energy generator 50 is transmitted to the electrode 60 of the handpiece through the connector and is applied to the skin surface that is in contact with the electrode 60.

The RF energy generator 50 of this embodiment generates RF energy having at least two or more different frequencies. That is, the RF energy generator 50 can selectively generate RF energy having a first frequency and RF energy having a second frequency. In this case, when the frequency range is divided into a first range (2~6MHz), a second range (6~10MHz), and a third range (10~30MHz), the first frequency may be a frequency within the first range and the second frequency may be a frequency within the second range. Alternatively, the first frequency may be a frequency within the first range and the second frequency may be a frequency within the third range. Alternatively, the first frequency may be a frequency within the second range and the second frequency may be a frequency within the third range. When the RF energy generator 50 can generate RF energy having three different frequencies, the first frequency may be a frequency within the first range, the second frequency may be a frequency within the second range, and the third frequency may be a frequency within the third range.

The RF energy generator 50, depending on a selected mode or the progress of a treatment process, may generate RF energy having any one selected frequency, or may generate RF energy combining RF pulses of a plurality of frequencies while controlling the ratio of the frequencies to be different.

A cooler 70 is a component that cools the electrode 60 of the handpiece. The cooler 70 may be configured in various cooling types, and, as an example, the cooler 70 of this embodiment is configured to cool the electrode 60 by delivering a refrigerant to the rear surface of the electrode 60. Accordingly, since the electrode 60 that is in contact with skin during treatment is cooled, thermal damage to the skin surface can be prevented during the transmission of RF energy.

In detail, the cooler 70 includes a refrigerant storage that accommodates a refrigerant, a cooling channel that forms a path through which the refrigerant accommodated in the refrigerant storage is delivered, and a cooling module 150 that sprays the refrigerant, which is delivered through the cooling channel, to the rear surface of the electrode 60 of the handpiece. The refrigerant storage is provided in the body or at a specific location. The cooling channel is connected from the refrigerant storage to the cooling module and at least a portion thereof is located inside the handpiece 20. Further, a valve that adjusts the amount of the refrigerant that is delivered is disposed on the path for delivering the refrigerant, and the on/off operations or the opening and closing amount of the valve can be controlled by a controller 40.

The cooler 70 can be configured to continuously spray a refrigerant to the rear surface of an electrode or to spray a refrigerant to the rear surface of an electrode in the form of pulses at intervals within a predetermined range by controlling a valve, or the like. The cooling performance of the cooler 70 may be adjusted by the amount of a refrigerant that is delivered to the rear surface of an electrode per unit time. The cooling performance may be adjusted by controlling the pressure inside the refrigerant storage, the on/off cycle of the valve, the opening and closing amount of the valve, or the like.

A sensing unit 80 is a component that senses various pieces of information for the operation of the treatment apparatus during or before/after treatment. For example, the sensing unit 80 may be at least one of an impedance sensor that measures the impedance of tissues, a temperature sensor that measures the temperature of an electrode or skin, a contact sensor that senses whether the electrode of the handpiece is in contact with a skin surface, and a movement sensor that senses the movement speed of the handpiece. As an example, a temperature sensor and a contact sensor may be disposed at positions adjacent to the electrode of the handpiece in this embodiment.

A storage unit 90 is a component that stores various pieces of information for treatment and includes a memory device. The storage unit 90 is disposed in the body 10 and may be additionally disposed in the handpiece 20 or a tip module 100. The storage unit 90 can store parameter information about each treatment mode, patient-related information, control information according to sensed conditions, etc. Further, the storage unit 90 may update and record sensed information and information input by a user during treatment. Further, when the handpiece 20 or the tip module 100 has a memory, the storage unit 90 may be configured to store the identification information of the handpiece or the tip module.

The controller 40 is a component that controls the operations of various components of the treatment apparatus such as the RF energy generator 50 and the cooler 70. For example, the controller 40 controls various components in various ways using settings configured by a user through a setting unit or control information stored in the storage unit. The controller receives sensed information from the sensing unit 80 and controls various components using the sensed information. For example, the controller 40 receives a temperature value sensed by a temperature sensor and controls the parameters of RF energy and cooling performance for an electrode on the basis of the temperature value. In this configuration, the controller 40 includes a computing unit and can compute real-time control values from sensing values using a preset algorithm and can control various components on the basis of the real-time control values.

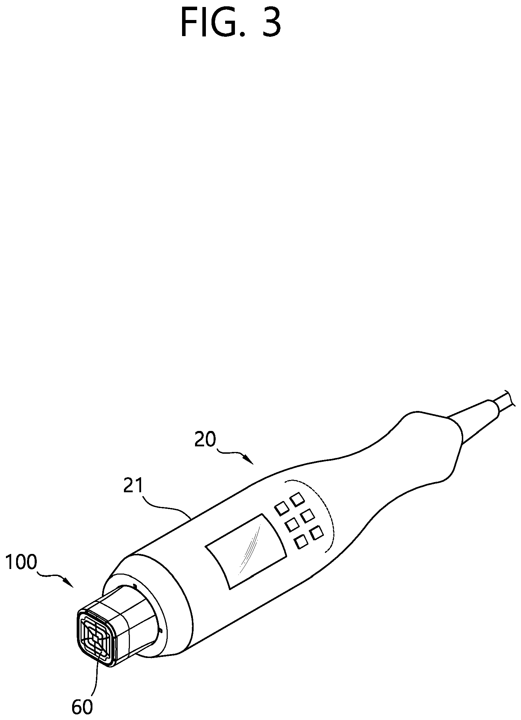

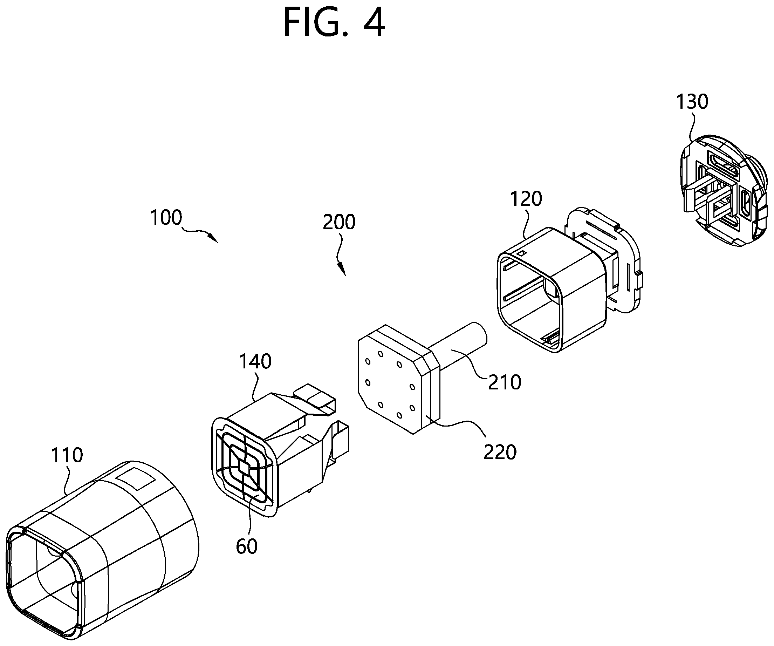

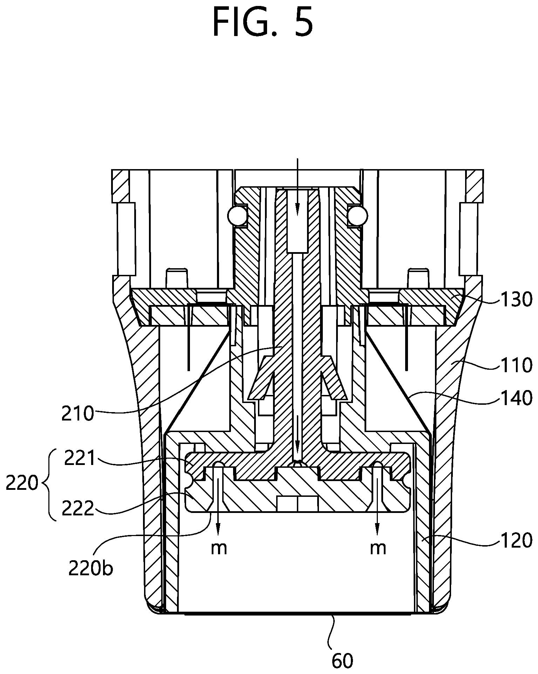

FIG. 3 is a perspective view showing the handpiece shown in FIG. 1. As shown in FIG. 3, the handpiece 20 includes a main body 21 and a tip module 100. An end of the main body 21 is connected with a connector, and various components for performing treatment operations such as an RF transmission circuit and a cooling channel are disposed in the main body 21. Controls and a display unit such as a display may be disposed on the outer surface of the main body 21. The tip module 100 has the electrode 60 for transmitting RF energy in contact with skin and is detachably coupled to an end of the main body 21. The tip module 100 has a circuit for transmitting RF energy to the electrode and a cooling structure for cooling the electrode. Hereafter, the structure of the tip module is described in more detail with reference to FIG. 4 and FIG. 5.

FIG. 4 is an exploded perspective view showing the main configuration of the tip module shown in FIG. 3 and FIG. 5 is a cross-sectional view showing the main configuration of the tip module shown in FIG. 3. Referring to FIG. 4 and FIG. 5, the tip module includes a tip housing 110, an electrode module 140, a cooling module 150, an internal case 120, and a rear cover 130.

The tip housing 110 and the internal case 120 are combined with each other, thereby supporting the electrode module 140. The cooling module 150 is disposed in the internal case 120. The rear cover 130 is coupled to the rear surface of the tip housing 110 with the electrode module 140, the internal case 120, and the cooling module 150 disposed in the tip housing 110. The tip housing 110 or the rear cover 130 has a coupling structure for fastening to the end portion of the main body 21 of the handpiece.

As shown in FIG. 4, the electrode module 140 is a flexible substrate that can be folded, and has electric devices and a circuit for electrically forming them. The electrode 60 is disposed on the front of the electrode module (when the electrode module is folded) and exposed through an opening of the tip housing 110 to come in contact with a skin surface. The electrode 60 includes a conductive layer formed on the flexible substrate and the conductive layer is configured to be covered by a dielectric layer. Accordingly, in treatment, the conductive layer of the electrode is brought in contact with skin through the dielectric layer, and when RF energy is applied to the electrode, the electrode transmits the RF energy to skin tissues in a capacitively coupled state to the skin tissues by the dielectric layer.

Meanwhile, as described above, the temperature sensor and the contact sensor of the sensing unit are disposed at positions adjacent to the electrode 60 of the electrode module 140, measure the temperature of the electrode or a skin surface, and sense whether the electrode comes in contact with skin. Further, the electrode module 140 further includes a memory and the memory can store information of the tip module such as the kind of the electrode, the size of the electrode, the pattern of the electrode, and the size of the cooling space. The electrode module 140 has a conductive lead that is connected with the electrode, the sensors, and the memory described above and extends rearward. A terminal formed at the end portion of the conductive lead is exposed rearward with the rear cover 130 coupled thereto and is electrically connected with the RF circuit at the main body 21 of the handpiece when the tip module 100 is coupled.

Further, the cooling module 150, as described above, is a component for spraying the refrigerant, which is delivered through the cooling channel from the refrigerant storage, to the rear surface of the electrode 60. A tube line 120 is disposed on the rear of the cooling module 200 and a plurality of spray holes 220b is disposed on the front of the cooling module 200. The rear end of the tube line 210 is exposed rearward through the rear cover 130 when the tip module 100 is assembled. Accordingly, when the tip module 100 is combined to the main body 21 of the handpiece, a cooling channel 220c of the tube line is coupled to the cooling channel at the main body 21, thereby forming a path through which the refrigerant provided from the refrigerant storage is delivered. This structure of the cooling module 200 will be described below in detail with reference to specific figures.

The tip module 100 having this structure, as described above, is detachably coupled to the end portion of the main body of the handpiece. When the tip module 100 is coupled, the controller 40 is provided with information of the tip module from the memory of the tip module 100 and controls the RF energy generator 50 and the cooler 70 in consideration of the information, thereby performing a process of transmitting RF energy to the electrode of the tip module 100 and cooling the electrode. Further, the tip module 100 may be a consumable and can be replaced with a new tip module 100 when a new patient is treated or the allowed number of uses is exceeded.

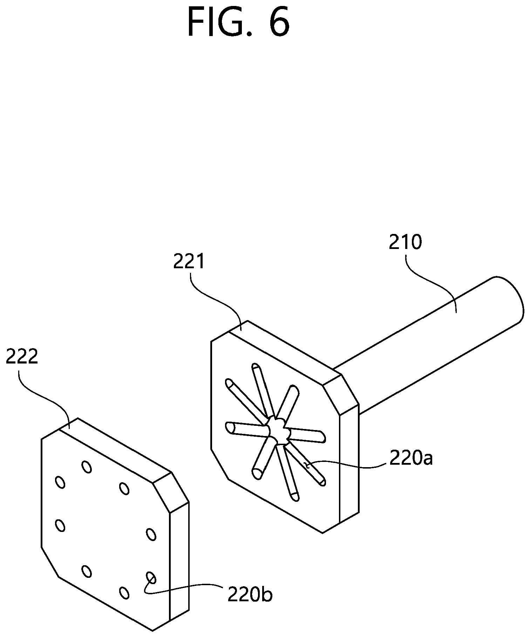

Hereafter, various embodiments of the cooling module are described in more detail with reference to FIG. 6 to FIG. 12.

FIG. 6 is a perspective view showing an embodiment of the cooling module shown in FIG. 4. As shown in FIG. 6, the cooling module 200 includes the tube line 210 described above and a cooling channel block 220.

As described above, the tube line 210 is disposed on the rear of the cooling module 200 and has one channel 220c therein through which a refrigerant is delivered (see FIG. 5). When the tip module is coupled, an end portion of the tube line 210 is coupled to the cooling channel of the main body 21 and forms a path through which a refrigerant is provided from the refrigerant storage.

The cooling channel block 220 includes a first block 222 and a second block 221. The first block 222 is disposed at the front (toward the electrode) and the second block 221 is disposed at the rear (toward the main body of the handpiece when the tip module is fastened), and the two blocks are combined with each other. The tube line 210 extends from the center of the rear of the second block 221. An opening connected with the cooling channel 220c of the tube line 210 is formed at the center of the second block 221 and grooves radially extending from the opening are formed on the front surface of the second block 221. The grooves form a plurality of branched channels 220a when they are coupled to the rear surface of the first block 222. The rear surface of the first block 222 may be a flat surface or grooves shaped to correspond to the grooves of the second block 221 may be formed on the rear surface of the first block 222. Further, the first block 222 has through-holes formed at the positions corresponding to the outer end portions of the grooves of the second block 221. Accordingly, the branched channels 220a form channels bending along the through-holes and a spray hole 220b is formed at each of the end portions of the channels 220a.

The refrigerant delivered through the cooling channel 220c of the tube line 210 splits into the plurality of branched channels 220a and is then discharged through the spray holes 220b. The plurality of branched channels 220a all have the same length, so a predetermined amount of refrigerant is uniformly sprayed at a predetermined pressure through each of the spray holes 220b.

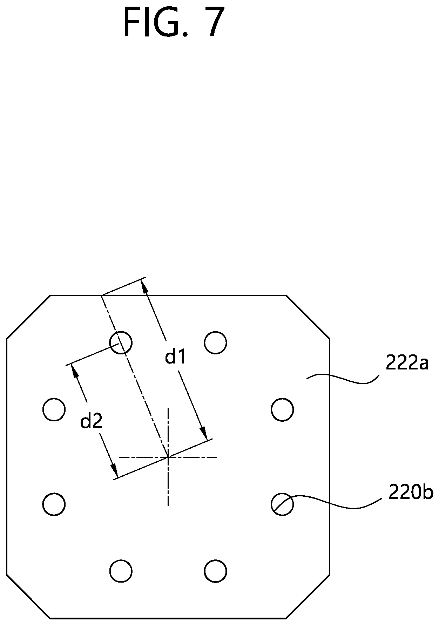

FIG. 7 is a plan view showing the front surface of the cooling module. As shown in FIG. 7, the plurality of spray holes 220b are circumferentially disposed around the center of the front surface 222a of the cooling module 200 and arranged at positions symmetric to each other. As an example, the plurality of spray holes 220b may be spaced at the same distance from the center of the front surface 222a and may be spaced at equal angles from each other. However, this is an example when the front surface of the electrode or the cooling module has a structure corresponding to a circle or a square, and when it has a structure corresponding to an ellipse or a rectangle, the plurality of spray holes may be symmetrically disposed in different ways.

Meanwhile, the spray holes 220b are disposed to be offset outward from the center on the front surface of the cooling module 200. In general, when RF energy is applied to skin tissues through an electrode, an edge effect phenomenon in which the current density concentrates on the edge rather than the center of the electrode occurs, so the surrounding of the electrode is relatively overheated. Accordingly, when the spray holes 220b are disposed to be offset to the outer edge, damage to skin due to an edge effect can be prevented. As an example, it is preferable the distance d2 at which the spray holes 220b are spaced from the center of the front surface is a half or more of the distance d1 from the center of the front surface to the outer edge passing through the spray holes.

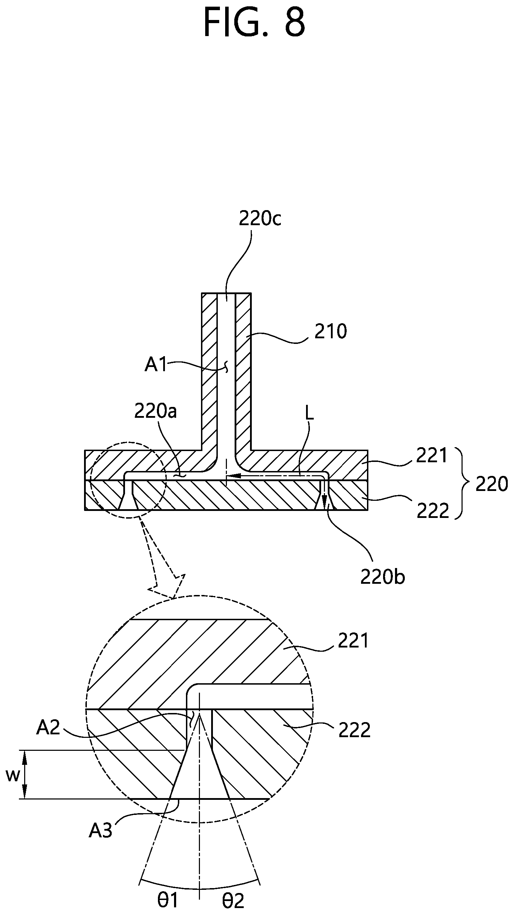

FIG. 8 is a cross-sectional view showing a cross-section of the cooling module according to an embodiment. Hereafter, the channel structure of the cooling module is described in more detail with reference to FIG. 8.

A cooling method according to this embodiment is a method of cooling the surface of an electrode or skin using the latent heat of vaporization of a refrigerant. The refrigerant delivered from the refrigerant storage is provided in a liquid state through the cooling channel, and is discharged through the spray holes and then vaporized, thereby cooling the rear surface of the electrode. Accordingly, the cooling channel is designed in consideration of the pressure and speed of the refrigerant passing through it so that the refrigerant can maintain a liquid state until it is discharged through the spray holes 220b. In this embodiment, the cross-section A1 of the cooling channel 220c of the tube line and the cross-section A2 of the branched channels 220a are designed in consideration of these conditions. In particular, the cross-section A2 of the branched channels 220a is configured to have an area smaller than the cross-sectional area A1 of the cooling channel so that the necessary pressure and speed can be maintained even when the refrigerant splits from one cooling channel 220c. As an example, the sum of the cross-sectional areas A2 of the plurality of branched channels may correspond to the cross-sectional area A1 of the cooling channel of the tube line.

However, when the cross-sectional area of the spray holes is small like the branched channels, the refrigerant is discharged at a high speed to have straightness. In order to overcome this limitation, the cooling module 200 of this embodiment is configured such that a widening section W is formed at each of the branched channels 220a at a position adjacent to the spray hole. The widening section W is a section in which the cross-sectional area and the diameter of the channel gradually increase in the flow direction of the refrigerant. Further, the spray hole 220b is formed at the end of the widening section W, whereby the spray hole 220b has a cross-sectional area A3 corresponding to the cross-sectional area of the end of the widening section. The refrigerant relatively decreases in speed and pressure as it passes through the widening section W, whereby the refrigerants discharged through the spray holes 220b have various directional components other than the straight direction. Accordingly, a refrigerant is intensively sprayed to a local portion of an electrode in a structure without a widening section, but a refrigerant can be more widely sprayed to the rear surface of an electrode due to the widening section W.

In detail, the widening section W is formed at a position adjacent to the spray hole on the bending channel provided in the first block 222 of the branched channel 220a. The inner wall of the widening section W forms an inclined surface inclined outward from the center axis of the channel in the flow direction of the refrigerant. As an example, the inner wall has a structure corresponding to the side of a frustum and has a predetermined inclination angle from the center axis of the channel (the center axis of the bending channel of the branched channel or the vertical axis of the cooling module) (Θ₁and Θ₂ are the same). In this case, the inclination angles (Θ₁, Θ₂) may be one value within the range of 10° to 80°. In detail, the inclination angles (Θ₁, Θ₂) may be one value within the range of 120° to 50°. The cross-sectional area A3 of the spray hole 220b is increased by the widening section W in comparison to the cross-sectional area A2 of the channel before the widening section. As an example, the cross-sectional area A3 of the spray hole 220b may have a size over 1.2 times, in more detail, over 2 times the cross-sectional area A2 of the channel before the widening section.

Accordingly, as compared with the related art, the refrigerant is sprayed with various directionality through the spray holes 220b, but, nevertheless, a relatively large amount of refrigerant reaches positions on the electrode 60 corresponding to the spray holes 220b, and the farther the distance from the corresponding positions, the less the refrigerant reaches. However, even though the distance increases, refrigerants sprayed through other spray holes 220b overlap as they reach toward the center of the electrode 60, so even though the spray holes 220b are positioned outward with respect to the electrode 60, it is possible to still cool down to the center of the electrode 60 relatively uniformly.

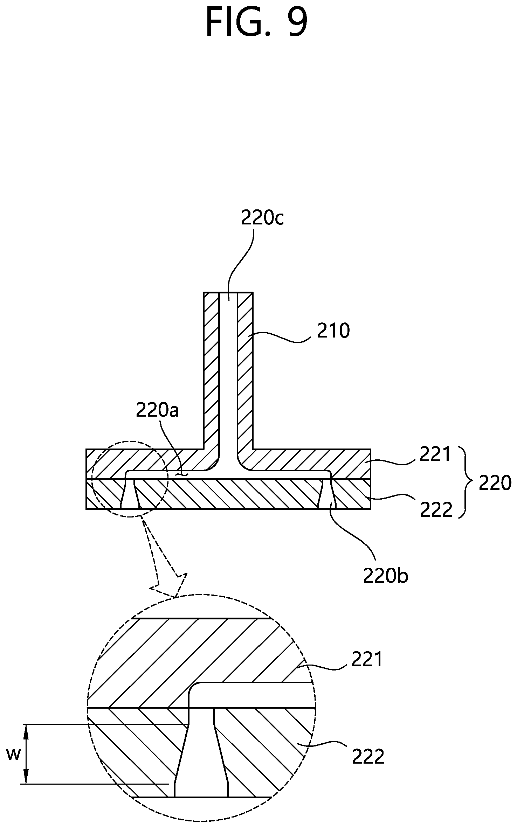

Meanwhile, the cooling module with a widening section may be modified and implemented in various ways other than the structure shown in FIG. 8. Hereafter, various embodiments of a cooling module with a widening section are described with reference to FIG. 9 to FIG. 12.

FIG. 9 is a cross-sectional view showing a cross-section of a cooling module according to another embodiment. In the embodiment of FIG. 8, the widening section has a structure in which the cross-sectional area of the channel gradually increases throughout the entire section. On the other hand, as in the embodiment shown in FIG. 9, the widening section W may be configured such that the diameter of the channel gradually increases in a partial section and the diameter of the channel remains constant in the other section adjacent to the spray hole.

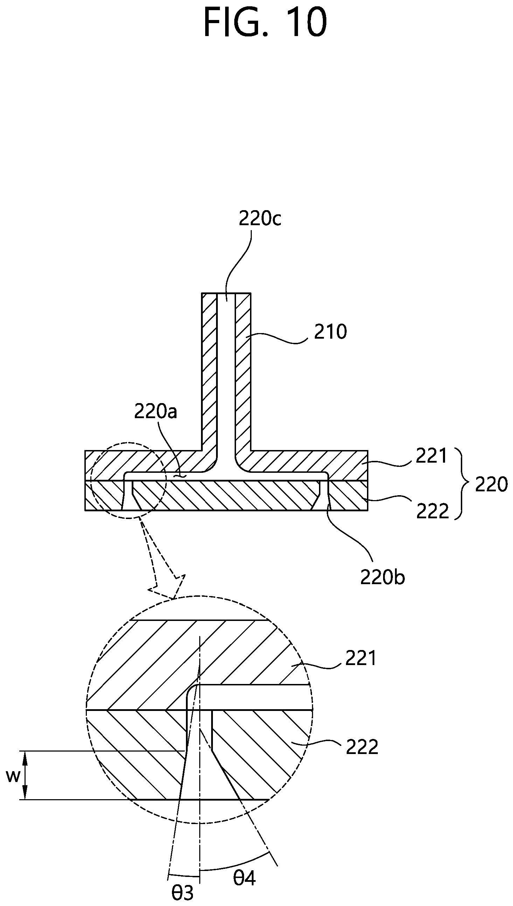

FIG. 10 is a cross-sectional view showing a cross-section of a cooling module according to another embodiment. In the embodiment of FIG. 8, the inner wall of the widening section W forms an inclined surface having a constant inclination angle around the center axis of the channel. However, as in the embodiment shown in FIG. 10, the inner wall of the channel of the widening section S may be configured to have different inclination angles along the circumference.

As shown in FIG. 10, the inner wall of the widening section W may be configured in the shape of the side of an inclined frustum. Accordingly, in the inner wall of the widening section W, the inner wall adjacent to the center of the front surface of the cooling module 200 forms a relatively large inclined surface and the inner wall adjacent to the outside forms a relatively small inclined surface (Θ₃ < Θ₄). In this case, the amount of the refrigerant sprayed outward and the directionality toward the outward direction are relatively limited, but the amount of the refrigerant sprayed to the center and the directionality toward the center can be increased. As described above, considering that the plurality of spray holes 220b are disposed to be offset outward with respect to the electrode 60, this embodiment has the advantage that it is possible to more uniformly cool down to the center of the electrode while sufficiently cooling the surrounding of the electrode to compensate for an edge effect.

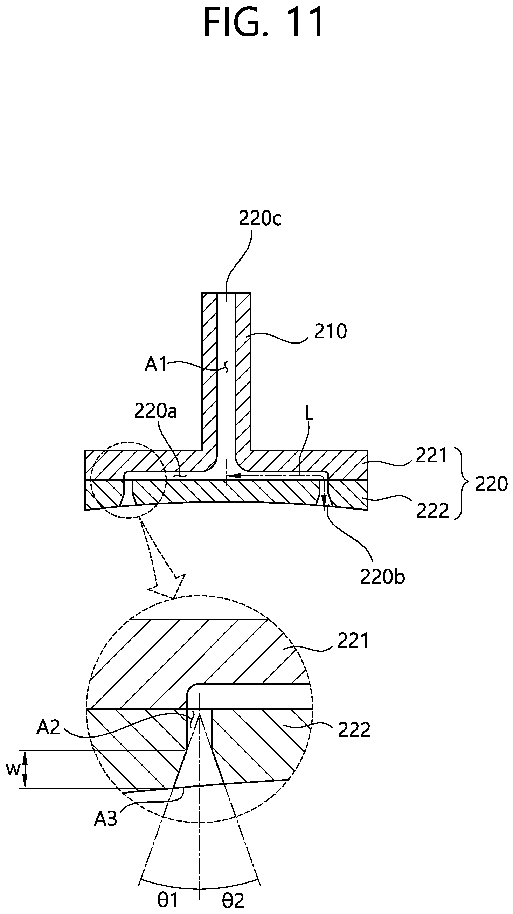

FIG. 11 is a cross-sectional view showing a cross-section of a cooling module according to another embodiment. In the case of the embodiment shown in FIG. 10, it is configured to relatively limit the outward flow of the refrigerant using the inclination angles of the inner wall of the widening section. On the other hand, as shown in FIG. 11, it is possible to achieve a similar objective by adjusting the gap from the electrode along their circumference.

The front surface of the cooling module on which the spray holes are formed is a flat surface in the above embodiments, but the front surface of the cooling module 200 according to this embodiment may be formed as a concave curved surface or a recessed stepped surface toward the center. Further, the spray holes 220b are formed on the curved or stepped surface. Accordingly, even though the inner wall of the widening section W has a constant angle from the center axis of the channel, the path length of the widening section changes along the circumference of each of the spray holes 220b. In detail, in the widening section W, the inner wall directed toward the center of the front surface forms a relatively shorter path than the inner wall directed outward, and the gap between the spray holes 220b and the electrode 60 is formed to be larger at the center side of the electrode than the edge side of the electrode. Accordingly, the refrigerant is discharged first inward through the spray holes before being discharged outward, so it is possible to limit spraying of the refrigerant toward the outer side of the electrode and guide the refrigerant to be sprayed toward the center.

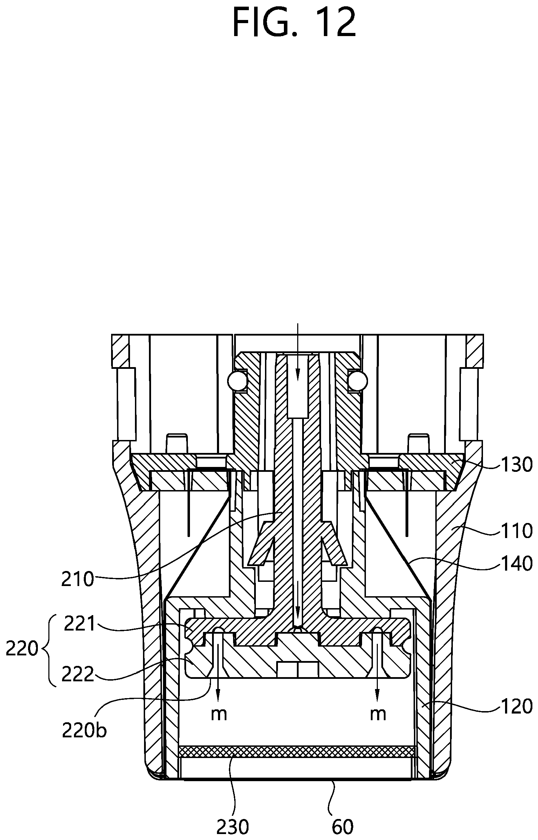

FIG. 12 is a cross-sectional view showing a cross-section of a cooling module according to another embodiment. The embodiment shown in FIG. 12, as compared with the embodiments shown in FIG. 8 to FIG. 11, further includes a distributing member 230 for uniformly distributing a refrigerant throughout the electrode. The distributing member may be a mesh member disposed in a cooling space between the cooling module 20 and the rear surface of the electrode 60. When the distributing member 230 is further provided, as described above, the refrigerants sprayed through the plurality of spray holes 220b are distributed through the distributing member, thereby being able to more uniformly cool the electrode 60.

The treatment field that uses a monopolar type of electrode was mainly described in the above embodiments, but the present disclosure is not limited thereto and can be applied to a treatment field that uses a bipolar type of electrode. Further, the aforementioned content can be applied to the field of treating skin tissues in various areas such as the face, neck, abdomen, and thighs using RF energy.

Embodiments of the present disclosure were described above in detail, but the present disclosure is not limited to the embodiments described above. It should be noted that those skilled in the art can change and modify the present disclosure in various ways without departing from the range of technical features defined in claims.

## Claims

1. A treatment apparatus using RF energy, comprising:
an electrode configured to transmit RF energy to tissues; and
a cooling module provided behind the electrode and including a plurality of branched channels, through which a refrigerant is delivered, and a plurality of spray holes provided at ends of the channels, respectively, to discharge the refrigerant,
wherein the branched channel has a shape of which a cross-sectional area increases in a flow direction of the refrigerant in a section adjacent to the spray hole.

2. The treatment apparatus of claim 1, wherein the section of the branched channel that is adjacent to the spray hole forms an inner wall inclined outward from a center axis of the channel.

3. The treatment apparatus of claim 2, wherein the inclined inner wall has an inclination angle within a range of 10° to 80° from the center axis of the channel.

4. The treatment apparatus of claim 1, wherein a cross-sectional area of the spray hole is 1.2 or more times a cross-sectional area of the channel before the section of which the cross-sectional area increases in the branched channel.

5. The treatment apparatus of claim 1, wherein a plurality of spray holes are provided on a surface of the cooling module and the plurality of spray holes are symmetrically disposed on the surface of the cooling module.

6. The treatment apparatus of claim 5, wherein at least four or more spray holes are provided on the surface of the cooling module.

7. The treatment apparatus of claim 1, wherein the plurality of spray holes are disposed at positions spaced a predetermined distance radially from a center of a surface of the cooling module and the spaced distance is a half or more of a distance from a center to an outer edge of the surface of the cooling module.

8. The treatment apparatus of claim 1, wherein the plurality of branched channels have the same length.

9. The treatment apparatus of claim 2, wherein the branched channel has a widening section formed such that a diameter increases in the flow direction of the refrigerant at a position adjacent to the spray hole, and
an inner wall forming the widening section is formed to have different inclination angles.

10. The treatment apparatus of claim 9, wherein, in the inner wall forming the widening section, the inner wall close to a center of a surface of the cooling module has a larger inclination angle than the inner wall far from the center of the surface of the cooling module.

11. The treatment apparatus of claim 1, wherein the spray holes are provided on a curved surface or a stepped surface of a front surface of the cooling module such that a distance from a rear surface of the electrode changes along a circumference of the spray holes, and
in the circumference of the spray holes, the distance from the rear surface of the electrode is larger at a portion positioned toward a center of the electrode than at a portion positioned toward an outer side of the electrode.

12. The treatment apparatus of claim 1, further comprising a distributing member disposed between the electrode and the cooling module and configured to distribute the refrigerant discharged through the spray holes.

13. A handpiece of a treatment apparatus using RF energy, the handpiece comprising:
an electrode configured to transmit RF energy to tissues; and
a cooling module provided behind the electrode and including a plurality of branched channels, through which a refrigerant passes, and spray holes provided at ends of the channels, respectively, to discharge the refrigerant,
wherein the branched channel has a shape of which a diameter increases in a flow direction of the refrigerant in a section adjacent to the spray hole.

14. A tip module of a treatment apparatus using RF energy, the tip module comprising:
an electrode configured to transmit RF energy to tissues; and
a cooling module provided behind the electrode and including a plurality of branched channels, through which a refrigerant passes, and spray holes provided at ends of the channels, respectively, to discharge the refrigerant,
wherein the branched channel has a shape of which a diameter increases in a flow direction of the refrigerant in a section adjacent to the spray hole.
